(19) Europäisches Patentamt
European Patent Office
Office européen des brevets

(11) Veröffentlichungsnummer: **0 563 817 A2**

(12) # EUROPÄISCHE PATENTANMELDUNG

(21) Anmeldenummer: **93105014.0**

(22) Anmeldetag: **26.03.93**

(51) Int. Cl.5: **C07C 205/45**, C07C 49/835, C07C 49/84, A01N 35/06, A01N 41/10, C07C 317/24

(30) Priorität: **31.03.92 DE 4210583**

(43) Veröffentlichungstag der Anmeldung: **06.10.93 Patentblatt 93/40**

(84) Benannte Vertragsstaaten: **DE FR GB IT**

(71) Anmelder: **HOECHST AKTIENGESELLSCHAFT**
**D-65926 Frankfurt(DE)**

(72) Erfinder: **Ort, Oswald, Dr.**
**Eppenhainer Strasse 14**
**W-6246 Glashütten 2(DE)**
Erfinder: **Willms, Lothar, Dr.**
**Lindenstrasse 17**
**W-5416 Hillscheid(DE)**
Erfinder: **Bauer, Klaus, Dr.**
**Doorner Strasse 53d**
**W-6450 Hanau(DE)**
Erfinder: **Bieringer, Hermann, Dr.**
**Eichenweg 26**
**W-6239 Eppstein/Ts(DE)**

(54) **Salze von 2-Benzoyl-cyclohexandionen, Selekfive herbizide Mittel, Verfahren zu ihrer Herstellung und ihre Verwendung zur Unkrautbekämpfung.**

(57) Verbindungen der Formel I,

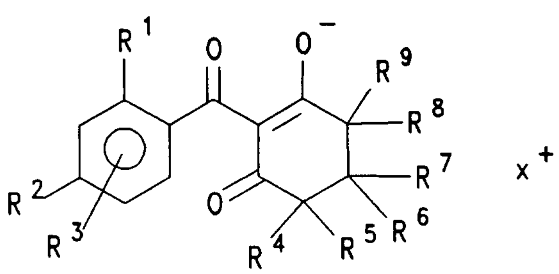

(I)

worin
$R^1$-$R^9$ und $x^+$ wie in Anspruch 1 definiert sind, eignen sich als selektive Herbizide, die im Vergleich zu den Verbindungen mit $x^+$ = Wasserstoff eine andere Wirkung aufweisen können, z.B. eine vorteilhafte Selektivität in Reiskulturen oder in Getreide und Mais.

EP 0 563 817 A2

Aus EP-A 137 963, EP-A 186 118, EP-A 274 634, EP-A 298 680 und US-4,780,127 sind 2-Benzoylcyclohexandion-derivate bekannt, die als Mittel zur Bekämpfung eines breiten Spektrums mono- und dikotyler Unkräuter beschrieben sind.

Aus WO 91/05470 sind 2-Benzoylcyclohexandionderivate bekannt, die bei niedrigen Wirkstoffdosierungen selektiv in Reispflanzen sind.

In JP-A-3063-248 sind Ammonium-Salze von 2-(2,4-Dichlorbenzoyl)cyclohexandion und 2-(2-Nitro-4-chlorbenzoyl)-cyclohexandion beschrieben, die in Reis bei Dosierungen von 32 bis 63 g ha eine bessere Selektivität aufweisen als 2-(2-Nitro-4-chlorbenzoyl)cyclohexandion. Bei Dosierungen über 63 g/ha weisen diese Ammoniumsalze eine hohe Phytotoxizität auf. Gleichzeitig wird eine breite Unkrautwirkung erst bei Dosierungen von 125 bis 250 g/ha erreicht.

Überraschenderweise wurde nun gefunden, daß Salze von speziell substituierten Cyclohexandionderivaten eine hervorragende Selektivität im Reis und eine ausgezeichnete Breitenwirkung gegen die in Reisarten typisch auftretenden Schadpflanzen aufweisen. Durch die hohe Selektivitätsspanne im Reis wird der Einsatz auch höherer Wirkstoffdosierungen ermöglicht, wodurch eine sehr breite Unkrautbekämpfung erzielt wird. Darüberhinaus wurde gefunden, daß diese Salze in anderen Kulturen wie Getreide und Mais sehr selektiv gegen einige wichtige Schadpflanzen eingesetzt werden können. Die zugrundeliegenden 2-Benzoylcyclohexandione sind dagegen in wichtigen Nutzkulturen wie Mais und Reis nicht ausreichend wirksam gegen die dort typisch vorkommenden Schadpflanzen, so daß in manchen Fällen erst synergistische Mischungen einen sinnvollen Einsatz der 2-Benzoylcyclohexandionderivate erlauben (vgl. EP-A-274 634; WO 91/05469).

Insbesondere werden durch die erfindungsgemäßen Verbindungen im Reisanbau vorkommende, oft schwer bekämpfbare perennierende Unkräuter, beispielsweise Sagittaria spec., Cyperus serotinus, Scirpus maritimus, Eleocharis spec. und Scirpus juncoides, sowie ein breites Spektrum annueller Unkräuter wirksam bekämpft.

Gegenstand der vorliegenden Erfindung sind deshalb Salze von 2-Benzoylcyclohexandionen der allgemeinen Formel I,

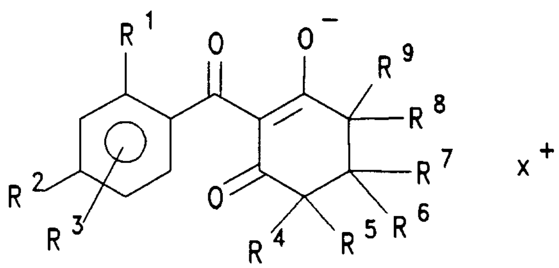

(I)

worin

| | |
|---|---|
| $R^1$ | Halogen, $(C_1\text{-}C_4)$-Alkoxy, $(C_1\text{-}C_4)$-Alkyl, $(C_1\text{-}C_4)$-Haloalkyl, $-NO_2$, -CN oder $S(O)_nR^{10}$; |
| $R^2$ und $R^3$ | unabhängig voneinander Wasserstoff, Halogen, $(C_1\text{-}C_4)$-Alkyl, $(C_1\text{-}C_4)$-Alkoxy, $(C_1\text{-}C_4)$Haloalkoxy, $(C_1\text{-}C_4)$-Haloalkyl, -CN, $-NO_2$, $-S(O)_m\text{-}R^{11}$,$-NR^{12}R^{13}$, $-NR^{14}\text{-CO-}R^{15}$, $-CO\text{-}R^{16}$; |
| $R^4$, $R^6$, $R^7$, $R^8$, $R^9$ | unabhängig voneinander Wasserstoff oder $(C_1\text{-}C_4)$-Alkyl; |
| $R^5$ | Wasserstoff, $(C_1\text{-}C_4)$-Alkyl oder $-CO\text{-}O\text{-}(C_1\text{-}C_4)$-Alkyl; |
| $R^{10}$ | $(C_1\text{-}C_4)$-Alkyl, $(C_1\text{-}C_4)$-Haloalkyl oder $(C_1\text{-}C_4)$-Alkoxy; |
| $R^{11}$ | $(C_1\text{-}C_4)$-Alkyl, $(C_1\text{-}C_4)$-Haloalkyl, Phenyl, Benzyl oder $-NR^{17}R^{18}$; |
| $R^{12}$ und $R^{13}$ | unabhängig voneinander Wasserstoff oder $(C_1\text{-}C_4)$-Alkyl; |
| $R^{14}$ | Wasserstoff oder $(C_1\text{-}C_4)$-Alkyl; |
| $R^{15}$ | $(C_1\text{-}C_4)$-Alkyl; |
| $R^{16}$ | Wasserstoff, $(C_1\text{-}C_4)$-Alkyl, $(C_1\text{-}C_4)$-Haloalkyl oder $(C_1\text{-}C_4)$-Alkoxy; |
| $R^{17}$ und $R^{18}$ | unabhängig voneinander Wasserstoff oder $(C_1\text{-}C_4)$-Alkyl und |
| n und m | unabhängig voneinander 0,1 oder 2 bedeuten, |
| und $x^+$ | ein Metallkation-Äquivalent, wie zum Beispiel ein Alkali- oder Erdalkali-Kationäquivalent, oder ein gegebenenfalls substituiertes Ammonium- oder Phosphonium-Ion der allgemeinen Formel II, |

$$\begin{array}{c} R^{20} \\ | \\ R^{19} - Y^{+} - R^{21} \\ | \\ R^{22} \end{array} \qquad (II)$$

worin Y für Stickstoff oder Phosphor steht,

$R^{19}$ Wasserstoff, $(C_1\text{-}C_{20})$Alkyl, $(C_2\text{-}C_{20})$Alkenyl oder $(C_1\text{-}C_{20})$Alkoxy, wobei letztere Reste gegebenenfalls ein- oder mehrfach durch Halogen, Alkoxy, Alkylthio, Mono- oder Dialkylamino substituiert sein können;

$R^{20}$, $R^{21}$ und $R^{22}$ unabhängig voneinander Wasserstoff, $(C_1\text{-}C_{20})$Alkyl, $(C_2\text{-}C_{20})$Alkenyl oder $(C_2\text{-}C_{20})$Alkinyl, wobei diese aliphatischen Reste ein- oder mehrfach durch Halogen, Amino, Alkylamino oder Dialkylamino substituiert oder ein- oder mehrfach durch Heteroatome oder Heteroatomgruppierungen wie Sauerstoff, Schwefel, -NH- oder $-N(C_1\text{-}C_{12})$alkyl unterbrochen sein können; Hydroxy-$(C_1\text{-}C_{20})$alkyl-, Hydroxy-$(C_2\text{-}C_{20})$-alkenyl- oder -alkinyl, $(C_1\text{-}C_{12})$Alkylsulfonyl-$(C_1\text{-}C_{12})$alkyl, $(C_3\text{-}C_{12})$-Cycloalkyl oder $(C_3\text{-}C_{12})$Cycloalkenyl, wobei diese cycloaliphatischen Reste ein oder mehrfach durch Halogen, Amino, Alkylamino oder Dialkylamino, $(C_1\text{-}C_{12})$-Alkylsulfonyl, Hydroxy oder $(C_1\text{-}C_{12})$Alkoxy substituiert und/oder ein- oder mehrfach durch Heteroatome bzw. Heteroatomgruppierungen wie Sauerstoff, Schwefel, -NH- oder $-N(C_1\text{-}C_{12})$alkyl unterbrochen sein können, oder einen Rest der Formel

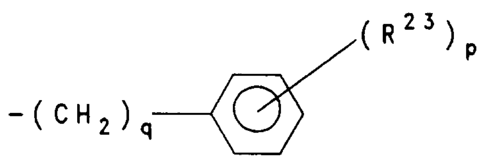

worin p und q 0,1 oder 2 und $R^{23}$ Wasserstoff, $(C_1\text{-}C_{12})$Alkyl, $(C_1\text{-}C_{12})$Alkoxy, $(C_1\text{-}C_{12})$Alkylthio, $(C_1\text{-}C_{12})$Alkylsulfonyl, CN, $NO_2$, Halogen, $(C_1\text{-}C_{12})$Haloalkyl, $(C_1\text{-}C_{12})$-Haloalkoxy, Amino, $(C_1\text{-}C_{12})$Alkylamino, $(C_1\text{-}C_{12})$Dialkylamino bedeuten,
oder die Reste $R^{20}$ und $R^{21}$ im Falle von Y = N zusammen mit dem Stickstoffatom ein cycloaliphatisches vier- bis zwölfgliedriges Ringssystem bilden, das gegebenenfalls ein- oder mehrfach durch Halogen, $(C_1\text{-}C_8)$Alkyl, $(C_3\text{-}C_8)$Alkenyl, $(C_1\text{-}C_8)$Alkoxy, Amino, $(C_1\text{-}C_8)$Alkylamino, $(C_1\text{-}C_8)$Dialkylamino, Phenyl, Benzyl oder Hydroxy substituiert und/oder ein- oder mehrfach durch Heteroatome bzw. Heteroatomgruppierungen wie Sauerstoff, Schwefel, -NH-oder $-N(C_1\text{-}C_{12})$alkyl unterbrochen ist und gegebenenfalls ein- oder mehrfach ungesättigt ist, bedeuten;
oder ein Sulfonium- bzw. Sulfoxoniumäquivalent der allgemeinen Formel III

$$\begin{array}{c} (O)_r \\ | \\ R^{19} - S^{+} - R^{21} \\ | \\ R^{20} \end{array} \qquad (III),$$

ist, worin $R^{19}$, $R^{20}$ und $R^{21}$ die oben angegebene Bedeutung haben und r für 0 oder 1 steht,

sowie deren tautomere Formen, mit Ausnahme solcher Verbindungen, in denen $R^1$ Chlor oder Nitro, $R^2$ Chlor und $R^3$ bis $R^9$ Wasserstoff bedeuten.

Unter Alkylresten werden Reste mit der angegebenen Anzahl Kohlenstoffatomen verstanden. Die Reste können geradkettig oder verzweigt sein. Die gebräuchlichsten Reste sind beispielsweise Methyl, Ethyl, n-Propyl, Isopropyl, n-Butyl, Isobutyl, sek.-Butyl oder tert.-Butyl. Unter Halogen wird Fluor, Chlor, Brom oder Jod verstanden. Haloalkyl-Reste können ein- oder mehrfach mit Halogen substituiert sein, d.h. auch perhalogeniert sein.

Bevorzugt sind Verbindungen der genannten Formel (I), worin

$R^1$ Fluor, Chlor, Brom, Jod, Methoxy, Nitro, Cyano oder $-S(O)_nR^{10}$;

$R^2$ und $R^3$ unabhängig voneinander Wasserstoff, Fluor, Chlor, Brom, Jod, Methyl, Methoxy, Trifluormethoxy, Difluormethoxy, Cyano, Nitro, Trifluormethyl, $-SO_2R^{11}$, $-NR^{12}R^{13}$, $-N(CH_3)-CO-R^{15}$ oder $-CO-O-(C_1-C_4)$-Alkyl und

$R^4$, $R^5$, $R^6$, $R^7$, $R^8$ und $R^9$ unabhängig voneinander Wasserstoff oder Methyl bedeuten und die übrigen Reste die angegebenen Bedeutungen haben.

Besonders bevorzugt sind Verbindungen der Formel (I), in denen

$R^2$ und $R^3$ unabhängig voneinander Wasserstoff, Fluor, Chlor, Brom, $-N(CH_3)_2$, Methoxy, Nitro, $-SO_2CH_3$, $-SO_2C_2H_5$, $-SO_2CH_2Cl$, $-SO_2N(CH_3)_2$, Trifluormethyl oder Difluormethoxy bedeuten.

Besonders geeignete erfindungsgemäße Verbindungen der Formel (I) sind die Verbindungen, in denen als Benzoylcyclohexandion-Komponente

2-(2-Chlor-4-methylsulfonylbenzoyl)-1,3-cyclohexandion,
2-(2-Chlor-4-ethylsulfonylbenzoyl)-1,3-cyclohexandion,
2-(2-Nitrobenzoyl)-4,4-dimethyl-1,3-cyclohexandion,
2-(2-Chlor-4-ethylsulfonylbenzoyl)-5,5-dimethyl-1,3-cyclohexandion,
2-(2-Nitro-4-chlorbenzoyl)-4-(1-methylethyl)-1,3-cyclohexandion,
2-(2-Nitro-4-chlorbenzoyl)-4,4-dimethyl-1,3-cyclohexandion,
2-(2-Chlor-4-methylsulfonylbenzoyl)-4,4-dimethyl-6-methyl-1,3-cyclohexandion,
2-(2-Nitro-4-methylsulfonylbenzoyl)-4,4-dimethyl-1,3-cyclohexandion,
2-(2-Nitro-4-difluormethoxybenzoyl)-1,3-cyclohexandion oder
2-(2-Nitro-4-difluormethoxybenzoyl)-4,4-dimethyl-1,3-cyclohexandion verwendet wird.

Die Verbindungen der Formel (I) können in verschiedenen tautomeren Strukturen (Keto-Enol Tautomerie) vorliegen, die Formel I beschreibt nur eine der möglichen Grenzformen:

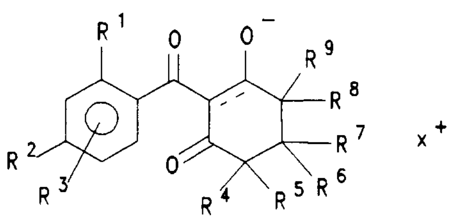

I

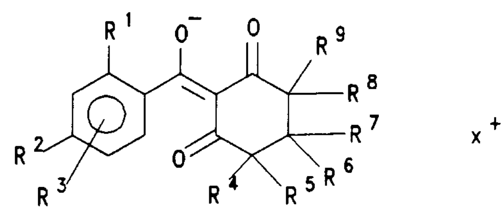

I a

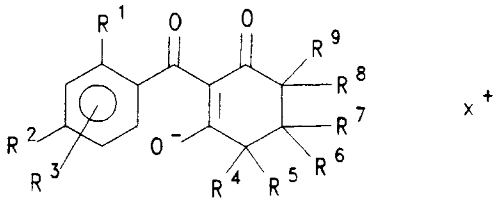

I b

Gegenstand der Erfindung ist ferner ein Verfahren zur Herstellung von Verbindungen der Formel I, das dadurch gekennzeichnet ist, daß man in Verbindungen der Formel IV

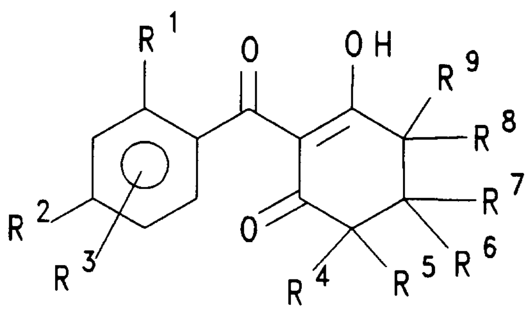

(IV)

die ebenfalls in verschiedenen tautomeren Strukturen vorliegen, das acide Wasserstoffatom durch ein für herbizide Anwendung geeignetes Kation der Formel $x^+$ ersetzt, indem man sie mit den dem Rest $x^+$ zugrundeliegenden anorganischen oder organischen Basen umsetzt.

Als Lösungsmittel verwendet man allgemein protische oder aprotische Lösungsmittel, wie z.B. Wasser, Methanol, Ethanol, Isopropanol, n-Butanol, Dimethylformamid, Dimethylsulfoxid, N-Methylpyrrolidon, Acetonitril, Aceton, Methylisobutyl-Keton, Dioxan, Tetrahydrofuran oder Gemische dieser Lösungsmittel und arbeitet bei Temperaturen zwischen -25 °C und der Rückflußtemperatur des Lösungsmittels, insbesondere zwischen 0 °C und 60 °C. Die Reihenfolge der Zugabe der Reaktanden ist nicht kritisch. Die Edukte werden im allgemeinen in stöchiometrischen Mengen je nach der Wertigkeit des Kations eingesetzt.

Geeignete Basen zur Herstellung der erfindungsgemäßen Salze sind beispielsweise Alkalicarbonate, wie Kaliumcarbonat, Alkali- und Erdalkalihydroxide, Ammoniak, Ethanolamin, Triethylamin, Diethylamin, Piperidin, Morpholin, Diethanolamin, Diallylamin, Tri-n-propylamin, Decylamin und Dodecylamin.

Die 2-Benzoylcyclohexandion-Derivate der Formel (I) sind bekannt, siehe z.B. die genannten EP-A-Veröffentlichungen 137 963, 186 118, 274 634 und 298 680; U.S.-Patent 4 780 127, WO 91/13548 und EP-Anmeldung 92103664.6, oder können nach den dort angegebenen Methoden hergestellt werden.

Gegenstand der vorliegenden Erfindung ist weiterhin ein Verfahren zur selektiven Bekämpfung von Schadpflanzen in Nutzpflanzenkulturen, wie Getreide, Mais und insbesondere in Reiskulturen, dadurch gekennzeichnet, daß man eine wirksame Menge einer oder mehrerer Verbindungen der allgemeinen Formel I auf die Anbaufläche, welche Schadpflanzen und Kulturpflanzen oder deren Samen enthält, appliziert. Geeignet sind z.B. Wirkstoffkonzentrationen von 0,001 bis 0,5 kg/ha, vorzugsweise 0,01 bis 0,25 kg/ha, insbesondere 0,02 bis 0,20 kg/ha, in Reiskulturen und 0,01 bis 2 kg/ha, vorzugsweise 0,05 bis 1,0 kg/ha in Getreide oder Mais.

Die erfindungsgemäß eingesetzten Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Bevorzugt ist die Anwendung im Vorauflauf- und/oder im frühen Nachauflaufverfahren. Im einzelnen seien beispielhaft einige Vertreter der mono- und dikotylen Unkrautflora genannt, die durch die erfindungsgemäßen Mittel kontrolliert werden können, ohne daß durch die Nennung eine Beschränkung auf bestimmte Arten erfolgen sollen.

Auf der Seite der monokotylen Unkrautarten werden z.B. Cyperusarten aus der annuellen Gruppe und auf seiten der perennierenden Spezies ausdauernde Cyperusarten, Scirpus-Arten und Eleocharis-Arten gut erfaßt.

Unter den spezifischen Kulturbedingungen im Reis vorkommende Unkräuter wie z.B. Sagittaria, Alisma, Eleocharis, Scirpus, Cyperus etc. werden von den erfindungsgemäß einzusetzenden Wirkstoffen hervorragend bekämpft.

Bei dikotylen Unkrautarten erstreckt sich das Wirkungsspektrum beispielsweise auf Rotala, Sphenoclea, Eclipta, Potamogeton, Heteranthera, Aeschynomene und Ammania.

Außerdem werden Unkrautarten erfaßt, die vorwiegend in anderen Kulturen wie Getreide und Mais auftreten: Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut

erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräuten.

Werden die erfindungsgemäß eingesetzten Verbindungen vor dem Keimen auf die Erdoberfläche appliziert, so wachsen die Unkräuter bis zum Keimblattstadium heran, stellen jedoch dann ihr Wachstum ein und sterben schließlich nach Ablauf von drei bis vier Wochen vollkommen ab.

Bei Applikation der Verbindungen der Formel (I) auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationspunkt vorhandenen Wuchsstadium stehen oder sterben nach einer gewissen Zeit ab, so daß auf diese Weise eine für die Kulturpflanzen schädliche Unkrautkonkurrenz sehr früh und nachhaltig durch den erfindungsgemäßen Einsatz der Verbindungen der Formel (I) beseitigt werden kann.

Obgleich die erfindungsgemäße eingesetzten Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Mais, Zuckerrübe, Baumwolle und Soja und insbesondere Reiskulturen aus gesätem oder verpflanztem Reis nur unwesentlich oder gar nicht geschädigt. Die Verbindungen der Formel (I) eignen sich aus diesen Gründen sehr gut zur selektiven Bekämpfung von Schadpflanzen landwirtschaftlichen Nutzpflanzungen, vorzugsweise in Getreide, Mais und Reis.

Die Aufwandmengen der Verbindungen der Formel (I) hängen von den Witterungs- und und Bodenverhältnissen sowie von den speziellen Schadpflanzen, den Kulturen und den jeweiligen Sorten, z.B. Reissorten ab. Besonders bevorzugt ist die Anwendung im späten Vorauflauf- oder frühen Nachauflaufverfahren.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), emulgierbare Konzentrate (EC), wäßrige Lösungen oder Konzentrate (SL), Emulsionen (EW) wie Öl- in Wasser- und Wasser-in-Öl-Emulsionen, versprühbare Lösungen, Kapselsuspensionen (CS), Dispersionen auf Öl- oder Wasserbasis, Suspoemulsionen, Suspensionskonzentrate (SC), Stäubemittel (DP), ölmischbare Lösungen (OL), Beizmittel, Granulate (GR) in Form von Mikro-, Sprüh-, Aufzugs- und Adsorptionsgranulaten, Granulate zur Boden- oder Streuapplikation, wasserdispergierbare Granulate (WG), ULV-Formulierungen, Mikrokapslen oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry"; 2nd Ed., J. Wiley & Sons, N.Y., Marsden, "Solvents Guide", 2nd Ed., Interscience, N.Y. 1950; McCutcheon's Detergents and Emulsifiers Annual", MG Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl., 1986.

Auf der Basis dieser Formulierungen lassen sich auch Kombinationen mit anderen pestizid wirksamen Stoffen, wie anderen Herbiziden, Fungiziden oder Insektiziden, oder auch Safenern, Düngemitteln und/oder Wachstumsregulatoren herstellen, z.B. in Form einer Fertigformulierung oder als Tankmix.

Spritzpulver (benetzbare Pulver) sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxalkylierte Fettalkohole oder polyoxyalkylierte Fettamine, Alkan- oder Alkylsulfonylbenzolsulfate und Dispergiermittel, z.B. ligninsulfonsaures Natrium, 2,2'-dinaphthylmethan-6,6'disulfonsaures Natrium, dibutylnaphthalin-sulfonsaures Natrium oder auch oleoylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:

Alkylarylsulfonsaures Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-fettsäureester oder Polyoxyethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophyllit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial.

Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen erhalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff, versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

A. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Freiheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösungmittel und 10 Gew.-Teilen oxethyliertes Nonylphenol als Emulgator.

e) Ein in Wasser dispergierbares Granulat wird erhalten indem man
75 Gewichtsteile Wirkstoff der Formel (I)
10 Gewichtsteile ligninsulfonsaures Calcium,
5 Gewichtsteile Natriumlaurylsulfat,
3 Gewichtsteile Polyvinylalkohol und
7 Gewichtsteile Kaolin
mischt, auf einer Stiftmühle mahlt und das Pulver in einem Wirbelbrett durch Aufsprühen von Wasser als Granulierflüssigkeit granuliert.

f) Ein in Wasser dispergierbares Granulat wird auch erhalten, indem man
25 Gewichtsteile Wirkstoff der Formel (I),
5 Gewichtsteile 2,2'-dinaphthylmethan-6,6'-disulfonsaures Natrium,
2 Gewichtsteile oleoylmethyltaurinsaures Natrium,
1 Gewichtsteil Polyvinylalkohol,
17 Gewichtsteile Calciumcarbonat und
50 Gewichtsteile Wasser
auf einer Kolloidmühle homogenisiert und vorzerkleinert, anschließend auf einer Perlmühle mahlt und die

so erhaltene Suspension in einem Sprühturm mittels einer Einstoffdüse zerstäubt und trocknet.

B. Chemische Beispiele

1. 2-(2-Chlor-4-methylsulfonylbenzoyl)-1,3-cyclohexandion-isopropylammonium-Salz
1,6 g (0,0049 mol) 2-(2-Chlor-4-methylsulfonyl)-1,3-cyclohexandion und 0,42 mol (0,048 mol) Isopropylamin werden in 20 ml Dichlormethan bei Raumtemperatur vereinigt und nach 2 Stunden eingedampft. Man erhält 1,9 g (100 % d.Th.) an 2-(2-Chlor-4-methylsulfonylbenzoyl)-1,3-cyclohexandion-isopropylammonium-Salz vom Schmelzpunkt 112 ° C.

2. 2-(2-Nitro-4-difluormethxy-benzoyl)-4,4-dimethyl-1,3-cyclohexandion-Natriumsalz
2,13 g (0,006 mol) 2-(2-Nitro-4-difluormethoxy-benzoyl)-4,4-dimethyl-1,3-cyclohexandion werden in 50 ml Methanol bei Raumtemperatur mit 0,24 g (0,006 mol) NaOH, in 5 ml $H_2O$ gelöst, vereinigt. Nach 1 Stunde wird eingedampft. Man erhält 2,35 g (99 % d.Th.) an 2-(2-Nitro-4-difluormethoxybenzoyl)-4,4-dimethyl-1,3-cyclohexandion-Natriumsalz vom Schmelzpunkt 160 ° C.

In analoger Weise werden die in Tabelle 1 aufgeführten Verbindungen erhalten:

Tabelle 1

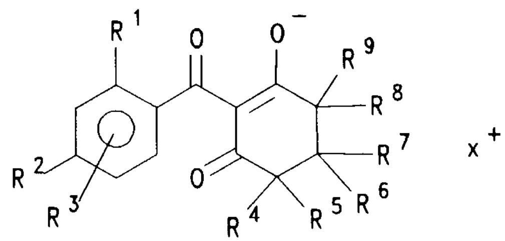

( I )

| Bsp. Nr. | $R^1$ | $R^2$ | $R^3$ | $R^4$ | $R^5$ | $R^6$ | $R^7$ | $R^8$ | $R^9$ | X | Schmp. [°C] |
|---|---|---|---|---|---|---|---|---|---|---|---|
| 1 | Cl | $CH_3SO_2$ | H | H | H | H | H | H | H | $(CH_3)_2CH\text{-}NH_3$ | 112 |
| 2 | $NO_2$ | $CF_2HO$ | H | $CH_3$ | $CH_3$ | H | H | H | H | Na | 160(Z) |
| 3 | $NO_2$ | $CF_2HO$ | H | $CH_3$ | $CH_3$ | H | H | H | H | K | 138 |
| 4 | $NO_2$ | $CF_2HO$ | H | $CH_3$ | $CH_3$ | H | H | H | H | $(CH_3)_2CH\text{-}NH_3$ | 114 |
| 5 | $NO_2$ | $CF_2HO$ | H | $CH_3$ | $CH_3$ | H | H | H | H | $(C_2H_5)_3NH$ | Oel |
| 6 | $NO_2$ | $CF_2HO$ | H | H | H | H | H | H | H | $(CH_3)_2CH\text{-}NH_3$ | 152 |
| 7 | $NO_2$ | $CF_2HO$ | H | H | H | H | H | H | H | $(C_2H_5)_3NH$ | 106 |

| Bsp. Nr. | $R^1$ | $R^2$ | $R^3$ | $R^4$ | $R^5$ | $R^6$ | $R^7$ | $R^8$ | $R^9$ | X | Schmp. [°C] |
|---|---|---|---|---|---|---|---|---|---|---|---|
| 8 | $NO_2$ | $CF_2HO$ | H | H | H | H | H | H | H | K | 140 |
| 9 | $NO_2$ | $CF_2HO$ | H | H | H | H | H | H | H | Na | 180 |
| 10 | Cl | $CH_3SO_2$ | H | H | H | H | H | H | H | $(C_2H_5)_3NH$ | 138 |
| 11 | Cl | $CH_3SO_2$ | H | H | H | H | H | H | H | Na | 164 |
| 12 | Cl | $CH_3SO_2$ | H | H | H | H | H | H | H | $(CH_3)_2CH)_2NH_2$ | 177 |
| 13 | $NO_2$ | $CH_3SO_2$ | H | H | H | H | H | H | H | $(C_2H_5)_3NH$ | |
| 14 | $NO_2$ | $CH_3SO_2$ | H | $CH_3$ | $CH_3$ | H | H | H | H | $(CH_3)_2CHNH_3$ | |
| 15 | $NO_2$ | $ClCH_2SO_2$ | H | H | H | H | H | H | H | $(CH_3)_2CHNH_3$ | |
| 16 | Cl | $CH_3SO_2$ | H | $CH_3$ | $CH_3$ | H | H | H | H | $n-C_4H_9NH_3$ | |
| 17 | Cl | $CH_3SO_2$ | H | $CH_3$ | H | $CH_3$ | H | H | H | $(CH_3)_2CHCH_2NH_3$ | |
| 18 | Cl | $CH_3SO_2$ | H | H | H | $CH_3$ | $CH_3$ | H | H | $t-C_4H_9NH_3$ | |
| 19 | F | $CH_3SO_2$ | H | H | H | H | H | H | H | $(C_2H_5)_3NH$ | |

| Bsp. Nr. | $R^1$ | $R^2$ | $R^3$ | $R^4$ | $R^5$ | $R^6$ | $R^7$ | $R^8$ | $R^9$ | X | Schmp. [°C] |
|---|---|---|---|---|---|---|---|---|---|---|---|
| 20 | Cl | $CH_3SO_2$ | 3-$OCH_3$ | H | H | H | H | H | H | $NH_4$ | |
| 21 | Cl | $CH_3SO_2$ | H | $(CH_3)_2CH$ | H | H | H | H | H | $(n\text{-}C_3H_7)_3NH$ | |
| 22 | Cl | $ClCH_2SO_2$ | H | H | H | H | H | H | H | 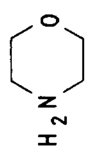 | |
| 23 | Br | $CH_3SO_2$ | 3-Br | H | H | H | H | H | H | Na | |
| 24 | Cl | $CH_3SO_2$ | H | $CH_3$ | $CH_3$ | H | H | $CH_3$ | H | K | |
| 25 | Cl | $CH_3SO_2$ | H | $CH_3$ | H | H | H | $CH_3$ | H | Na | |
| 26 | Cl | $CH_3SO_2$ | H | $CH_3$ | $CH_3$ | H | H | $CH_3$ | $CH_3$ | $NH_4$ | |
| 27 | Cl | $C_2H_5SO_2$ | H | $CH_3$ | $CH_3$ | H | H | H | H | $C_2H_5NH_3$ | |
| 28 | Cl | $nC_3H_7SO_2$ | H | H | H | H | H | H | H | $(C_2H_5)_3NH$ | |

C. Biologische Beispiele

1. Unkrautwirkung im Vorauflauf

Samen bzw. Rhizomstücke von mono- und dikotylen Unkrautpflanzen wurden in Plastiktöpfen in sandiger

Lehmerde ausgelegt und mit Erde abgedeckt. Die in Form von benetzbaren Pulvern oder Emulsionskonzentraten formulierten Wirkstoffe der Formel (I) wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert. Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach 3 bis 4 Wochen im Vergleich zu unbehandelten Kontrollen.

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

Die erfindungsgemäßen eingesetzten Wirkstoffe wiesen eine gute herbizide Vorauflaufwirksamkeit gegen ein breites Spektrum von Ungräsern und Unkräutern auf, wobei die Reispflanzen wenig oder gar nicht geschädigt wurden. Einige Ergebnisse sind in Tabelle 2 zusammengestellt.

Tabelle 2

| Wirkung im Vorauflauf | | | | | | | |
|---|---|---|---|---|---|---|---|
| Bsp.-Nr. | Dosis (kg a.i./ha) | Herbizide Wirkung | | | | | |
| | | STME | CHSE | SIAL | LOMU | ECCR | AVSA |
| 1 | 1,25 | 5 | 5 | 5 | 5 | 5 | 4 |
| 2 | 1,25 | 5 | 5 | 5 | 5 | 5 | 5 |
| 3 | 1,25 | 5 | 5 | 5 | 5 | 5 | 4 |
| 4 | 1,25 | 5 | 5 | 5 | 5 | 5 | 5 |
| 5 | 1,25 | 5 | 5 | 5 | 5 | 5 | 5 |
| 6 | 1,25 | 5 | 5 | 5 | 5 | 5 | 5 |
| 7 | 1,25 | 5 | 5 | 5 | 5 | 5 | 5 |
| 8 | 1,25 | 5 | 5 | 5 | 5 | 5 | 4 |
| 9 | 1,25 | 5 | 5 | 5 | 5 | 5 | 5 |
| 10 | 1,25 | 5 | 5 | 5 | 5 | 5 | 5 |
| 11 | 1,25 | 5 | 5 | 5 | 5 | 5 | 5 |
| 12 | 1,25 | 5 | 5 | 5 | 5 | 5 | 5 |

Abkürzungen:
STME = Stellaria media
CHSE = Chrysanthemum segetum
SIAL = Sinapis alba
LOMU = Lolium multiflorum
ECCR = Echinochloa crus-galli
AVSA = Avena sativa
a.i. = Aktivsubstanz

2. Unkrautwirkung im Nachauflauf

Samen bzw. Rhizomstücke von monokotylen und dicotylen Unkrautpflanzen wurden in Plastiktöpfen in sandiger Lehmerde ausgelegt und im Gewächshaus unter guten Wachstumsbedingungen herangezogen. Drei Wochen nach der Aussaat werden die Versuchspflanzen im Dreiblattstadium behandelt.

Die als Spritzpulver bzw. als Emulsionskonzentrate formulierten Wirkstoffe der Formel (I) werden in verschiedenen Dosierungen mit einer Wasseraufwandmenge von umgerechnet 300 bis 600 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel wiesen auch im Nachauflauf eine gute herbizide Wirksamkeit gegen ein breites Spektrum wirtschaftlich wichtiger Ungräser und Unkräuter auf (vgl. Tabelle 3).

Tabelle 3

| Nachlaufwirkung | | | | | |
|---|---|---|---|---|---|
| Bsp.-Nr. | Dosis (kg a.i./ha) | Herbizide Wirkung | | | |
| | | STME | CHSE | SIAL | ECCR |
| 1 | 1,25 | 5 | 5 | 5 | 5 |
| 2 | 1,25 | 5 | 5 | 5 | 4 |
| 3 | 1,25 | 5 | 5 | 5 | 5 |
| 4 | 1,25 | 4 | 5 | 5 | 5 |
| 5 | 1,25 | 5 | 5 | 5 | 4 |
| 6 | 1,25 | 5 | 5 | 5 | 5 |
| 7 | 1,25 | 4 | 5 | 5 | 5 |
| 8 | 1,25 | 5 | 5 | 5 | 5 |
| 9 | 1,25 | 5 | 5 | 5 | 5 |
| 10 | 1,25 | 5 | 5 | 5 | 5 |
| 11 | 1,25 | 5 | 5 | 5 | 5 |
| 12 | 1,25 | 5 | 5 | 5 | 5 |

3. Unkrautwirkung und Selektivität im Reis

Speziell im Reisanbau vorkommende Unkräuter sowie Reispflanzen wurden in mit Wasser gesättigten Boden kultiviert. Die in Form von benetzbaren Pulvern oder Emulsionskonzentraten formulierten Wirkstoffe der Formel (I) wurden dann als wäßrige Suspensionen bzw. Emulsionen mit einer Wasseraufwandmenge von umgerechnet 600 bis 800 l/ha in unterschiedlichen Dosierungen darüber appliziert. Nach der Behandlung wurden die Töpfe im Gewächshaus aufgestellt. Die optische Bonitur erfolgte nach dem Auflaufen der Versuchspflanzen nach 3 bis 4 Wochen im Vergleich zu unbehandelten Kontrollen.

Die erfindungsgemäßen eingesetzten Wirkstoffe wiesen eine gute herbizide Vorauflaufwirksamkeit gegen ein breites Spektrum von Ungräsern und Unkräutern auf, wobei die Reispflanzen wenig oder gar nicht geschädigt wurden. Einige Ergebnisse sind in Tabelle 4 zusammengestellt.

Tabelle 4

| Produkt Nr. | Dosis (kg a.i./ha) | herbizide Wirkung | | | |
|---|---|---|---|---|---|
| | | SAPY | ELAC | CYMO | ORSA-V |
| 1 | 0,25<br>0,12 | 5<br>5 | 5<br>4 | 5<br>5 | 1<br>0 |
| 2 | 0,25<br>0,12 | 5<br>5 | 5<br>5 | 5<br>5 | 1<br>0 |
| 6 | 0,25<br>0,12 | 5<br>5 | 5<br>4 | 5<br>5 | 1<br>0 |
| 8 | 0,25<br>0,12 | 5<br>5 | 5<br>5 | 5<br>5 | 1<br>0 |
| 9 | 0,25<br>0,12 | 5<br>5 | 5<br>4 | 5<br>5 | 2<br>0 |
| 10 | 0,25<br>0,12 | 5<br>5 | 5<br>4 | 5<br>5 | 1<br>0 |
| 11 | 0,25<br>0,12 | 5<br>5 | 5<br>4 | 5<br>5 | 0<br>0 |
| 12 | 0,25<br>0,12 | 5<br>5 | 5<br>4 | 5<br>5 | 1<br>0 |
| Abkürzungen:<br>SAPY = Sagittaria pygmaea<br>ELAC = Eleocharis acicularis<br>CYMO = Cyperus monti (= serotinus)<br>ORSA-V = Oryza sativa (verpflanzt) | | | | | |

## Patentansprüche

**1.** Salze von 2-Benzoylcyclohexandionen der allgemeinen Formel I,

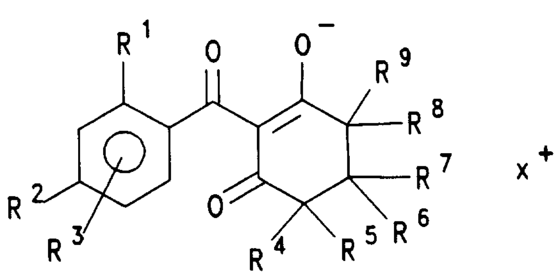

(I)

worin

| | |
|---|---|
| $R^1$ | Halogen, $(C_1$-$C_4)$-Alkoxy-, $(C_1$-$C_4)$-Alkyl, $(C_1$-$C_4)$-Haloalkyl, $-NO_2$, -CN oder S-$(O)_nR^{10}$; |
| $R^2$ und $R^3$ | unabhängig voneinander Wasserstoff, Halogen, $(C_1$-$C_4)$-Alkyl, $(C_1$-$C_4)$-Alkoxy, $(C_1$-$C_4)$Haloalkoxy, $(C_1$-$C_4)$-Haloalkyl, -CN, $-NO_2$, $-S(O)_m$-$R^{11}$,$-NR^{12}R^{13}$, $-NR^{14}$-CO-$R^{15}$, -CO-$R^{16}$; |
| $R^4$, $R^6$, $R^7$, $R^8$, $R^9$ | unabhängig voneinander Wasserstoff oder $(C_1$-$C_4)$-Alkyl; |
| $R^5$ | Wasserstoff, $(C_1$-$C_4)$-Alkyl oder -CO-O-$(C_1$-$C_4)$-Alkyl; |
| $R^{10}$ | $(C_1$-$C_4)$-Alkyl, $(C_1$-$C_4)$-Haloalkyl oder $(C_1$-$C_4)$-Alkoxy; |

$R^{11}$ $(C_1\text{-}C_4)$-Alkyl, $(C_1\text{-}C_4)$-Haloalkyl, Phenyl, Benzyl oder $-NR^{17}R^{18}$;
$R^{12}$ und $R^{13}$ unabhängig voneinander Wasserstoff oder $(C_1\text{-}C_4)$-Alkyl;
$R^{14}$ Wasserstoff oder $(C_1\text{-}C_4)$-Alkyl;
$R^{15}$ $(C_1\text{-}C_4)$-Alkyl;
$R^{16}$ Wasserstoff, $(C_1\text{-}C_4)$-Alkyl, $(C_1\text{-}C_4)$-Haloalkyl oder $(C_1\text{-}C_4)$-Alkoxy;
$R^{17}$ und $R^{18}$ unabhängig voneinander Wasserstoff oder $(C_1\text{-}C_4)$-Alkyl und
n und m unabhängig voneinander 0,1 oder 2 bedeuten,
und $x^+$ ein Metallkation-Äquivalent, oder ein gegebenenfalls substituiertes Ammonium- oder Phosphonium-Ion der allgemeinen Formel II,

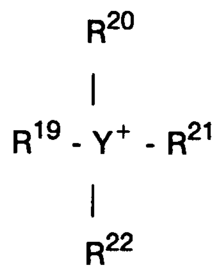

(II)

worin Y für Stickstoff oder Phosphor steht,

$R^{19}$ Wasserstoff, $(C_1\text{-}C_{20})$Alkyl, $(C_1\text{-}C_2)$Alkenyl oder $(C_1\text{-}C_{20})$Alkoxy, wobei letztere Reste gegebenenfalls ein- oder mehrfach durch Halogen, Alkoxy, Alkylthio, Mono- oder Dialkylamino substituiert sein können;

$R^{20}$, $R^{21}$ und $R^{22}$ unabhängig voneinander Wasserstoff, $(C_1\text{-}C_{20})$Alkyl, $(C_1\text{-}C_{20})$Alkenyl oder $(C_1\text{-}C_{20})$Alkinyl, wobei diese aliphatischen Reste ein- oder mehrfach durch Halogen, Amino, Alkylamino oder Dialkylamino substituiert oder ein- oder oder mehrfach durch Heteroatome oder Heteroatomgruppierungen unterbrochen sein können; Hydroxy-$(C_1\text{-}C_{20})$alkyl-, -alkenyl- oder alkinyl, $(C_1\text{-}C_{12})$Alkylsulfonyl-$(C_1\text{-}C_{12})$alkyl, $(C_3\text{-}C_{12})$Cycloalkyl oder $(C_3\text{-}C_{12})$Cycloalkenyl, wobei diese cycloaliphatischen Reste ein oder mehrfach durch Halogen, Amino, Alkylamino oder Dialkylamino, $(C_1\text{-}C_{12})$Alkylsulfonyl, Hydroxy oder $(C_1\text{-}C_{12})$Alkoxy substituiert und/oder ein- oder mehrfach durch Heteroatome bzw. Heteroatomgruppierungen unterbrochen sein können, oder einen Rest der Formel

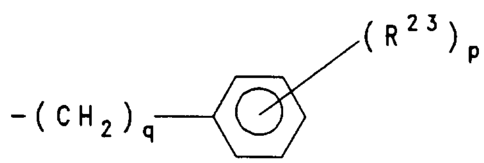

worin p und q 0,1 oder 2 und $R^{23}$ Wasserstoff, $(C_1\text{-}C_{12})$Alkyl, $(C_1\text{-}C_{12})$Alkoxy, $(C_1\text{-}C_{12})$Alkylthio, $(C_1\text{-}C_{12})$Alkylsulfonyl, CN, $NO_2$, Halogen, $(C_1\text{-}C_{12})$Haloalkyl, $(C_1\text{-}C_{12})$Haloalkoxy, Amino, $(C_1\text{-}C_{12})$Alkylamino, $(C_1\text{-}C_{12})$Dialkylamino bedeuten, oder die Reste $R^{20}$ und $R^{21}$ im Falle von Y = N zusammen mit dem Stickstoffatom ein cycloaliphatisches vier- bis zwölfgliedriges Ringssystem bilden, das gegebenenfalls ein- oder mehrfach durch Halogen, $(C_1\text{-}C_8)$Alkyl, $(C_3\text{-}C_8)$Alkenyl, $(C_1\text{-}C_8)$Alkoxy, Amino, $(C_1\text{-}C_8)$Alkylamino, $(C_1\text{-}C_8)$Dialkylamino, Phenyl, Benzyl oder Hydroxy substituiert und/oder ein- oder mehrfach durch Heteroatome bzw. Heteroatomgruppierungen unterbrochen ist und gegebenenfalls ein- oder mehrfach ungesättigt ist, bedeuten;

oder ein Sulfonium- bzw. Sulfoxoniumäquivalent der allgemeinen Formel III

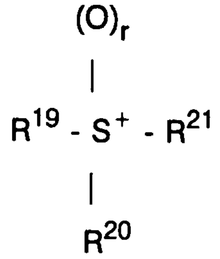

(III),

ist, worin $R^{19}$, $R^{20}$ und $R^{21}$ die oben angegebene Bedeutung haben und r für 0 oder 1 steht,

sowie deren tautomere Formen, mit Ausnahme solcher Verbindungen, in denen $R^1$ Chlor oder Nitro, $R^2$ Chlor und $R^3$ bis $R^9$ Wasserstoff bedeuten.

**2.** Verbindungen der Formel (I) nach Anspruch 1, dadurch gekennzeichnet, daß

| | |
|---|---|
| $R^1$ | Fluor, Chlor, Brom, Jod, Methoxy, Nitro, Cyano oder $-S(O)_nR^{10}$; |
| $R^2$ und $R^3$ | unabhängig voneinander Wasserstoff, Fluor, Chlor, Brom, Jod, Methyl, Methoxy, Trifluormethoxy, Difluormethoxy, Cyano, Nitro, Trifluormethyl, $-SO_2R^{11}$, $-NR^{12}R^{13}$, $-N(CH_3)-CO-R^{15}$ oder $-CO-O-(C_1-C_4)$-Alkyl und |
| $R^4$, $R^5$, $R^6$, $R^7$, $R^8$ und $R^9$ | unabhängig voneinander Wasserstoff oder Methyl bedeuten. |

**3.** Verbindungen der Formel (I) nach Anspruch 1 oder 2, dadurch gekennzeichnet, daß

$R^2$ und $R^3$ unabhängig voneinander Wasserstoff, Fluor, Chlor, Brom, $-N(CH_3)_2$, Methoxy, Nitro, $-SO_2CH_3$, $-SO_2C_2H_5$, $-SO_2CH_2Cl$, $-SO_2N(CH_3)_2$, Trifluormethyl oder Difluormethoxy bedeuten.

**4.** Verfahren zur Herstellung von Verbindungen der Formel I gemäß Anspruch 1, das dadurch gekennzeichnet ist, daß man in Verbindungen der Formel IV

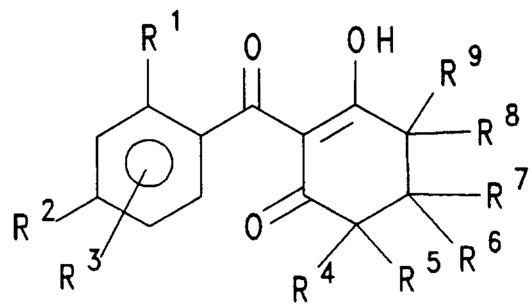

(IV)

das acide Wasserstoffatom durch ein für herbizide Anwendung geeignetes Kation der Formel $x^+$ ersetzt, indem man sie mit den dem Rest $x^+$ zugrundeliegenden anorganischen oder organischen Basen umsetzt.

**5.** Verfahren nach Anspruch 4, dadurch gekennzeichnet, daß als Verbindung der Formel IV eine der nachstehenden Verbindungen verwendet wird:

2-(2-Chlor-4-methylsulfonylbenzoyl)-1,3-cyclohexandion,
2-(2-Chlor-4-ethylsulfonylbenzoyl)-1,3-cyclohexandion,
2-(2-Nitrobenzoyl)-4,4-dimethyl-1,3-cyclohexandion,
2-(2-Chlor-4-ethylsulfonylbenzoyl)-5,5-dimethyl-1,3-cyclohexandion,
2-(2-Nitro-4-chlorbenzoyl)-4-(1-methylethyl)-1,3-cyclohexandion,
2-(2-Nitro-4-chlorbenzoyl)-4,4-dimethyl-1,3-cyclohexandion,
2-(2-Chlor-4-methylsulfonylbenzoyl)-4,4-dimethyl-6-methyl-1,3-cyclohexandion,
2-(2-Nitro-4-methylsulfonylbenzoyl)-4,4-dimethyl-1,3-cyclohexandion,

2-(2-Nitro-4-difluormethoxybenzoyl)-1,3-cyclohexandion oder
2-(2-Nitro-4-difluormethoxybenzoyl)-4,4-dimethyl-1,3-cyclohexandion.

**6.** Mittel zur selektiven Bekämpfung von Schadpflanzen in Nutzpflanzungen, gekennzeichnet durch einen selektiv wirksamen Gehalt an einer Verbindung der Formel I nach Anspruch 1, 2 oder 3 und einem Gehalt an im Pflanzenschutz üblichen Formulierungshilfsmitteln.

**7.** Verfahren zur Bekämpfung von Schadpflanzen in Nutzpflanzungen, dadurch gekennzeichnet, daß man eine oder mehrere Verbindungen der Formel I nach einem der Ansprüche 1 bis 3 in einer Menge, die für die Erzielung einer selektivherbiziden Wirkung geeignet ist, auf die Anbaufläche aufbringt.

**8.** Verfahren gemäß Anspruch 7, dadurch gekennzeichnet, daß man die Verbindungen der Formel I in einer Konzentration von 0,001 bis 0,5 kg/ha in Reiskulturen anwendet.

**9.** Verfahren gemäß Anspruch 7, dadurch gekennzeichnet, daß man die Verbindungen der Formel I in einer Konzentration von 0,01 bis 2 kg/ha in Getreide- oder Maiskulturen anwendet.

**10.** Verwendung von Verbindungen der Formel I nach einem der Ansprüche 1 bis 3 als Herbizide zur Bekämpfung von Schadpflanzen in Nutzpflanzenkulturen.